# EUROPEAN PATENT SPECIFICATION

(11) **EP 0 579 614 B1**
(45) Date of publication and mention of the grant of the patent: **28.05.1997**
(21) Application number: 92905201.7
(22) Date of filing: 28.02.1992
(51) Int. Cl.: C07D 471/06, A61K 31/435

(54) **IMIDAZOACRIDINES AND THEIR ANTINEOPLASTIC USE**
IMIDAZOACRIDINE UND IHRE ANTINEOPLASTISCHE ANWENDUNG
IMIDAZOACRIDINES ET LEUR UTILISATION EN TANT QU'ANTINEOPLASIQUE

(30) Priority: 05.03.1991 GB 9104548
(43) Date of publication of application: 26.01.1994
(73) Proprietor: BRITISH TECHNOLOGY GROUP LTD, London SE1 6BU (GB)
(72) Inventor: CHOLODY, Wieslaw, Marek, 84-200 Wejherowo (PL); KONOPA, Jerzy, Kazimierz, 80-809 Gdansk (PL)
(74) Representative: Percy, Richard Keith
(86) International application number: PCT/GB92/00360
(87) International publication number: WO 92/15583

(56) References cited:
- JOURNAL OF MEDICINAL CHEMISTRY. vol. 33, no. 1, January 1990, WASHINGTON US pages 49 - 52; W. M. CHOLODY ET AL.: "5-[(Aminoalkyl)amino]imid azo[4,5,1- de]acridin-6-ones as a Novel Class of Antineoplastic Agents. Synthesis and Biological Activity"
- JOURNAL OF MEDICINAL CHEMISTRY. vol. 33, no. 10, October 1990, WASHINGTON US pages 2852 - 2856; W. M. CHOLODY ET AL.: "8-Substituted 5-[(Aminoal kyl)amino]-6H-v-triazolo[4,5,1-de]acridin-6-ones as potential antineoplastic agents. Synthesis and biological activity"
- JOURNAL OF MEDICINAL CHEMISTRY. vol. 35, no. 1, 24 January 1992, WASHINGTON US pages 378 - 382; W. M. CHOLODY ET AL.: 'Chromophore-Modified Antineoplastic Imidazoacridinones. Synthesis and Activity against Murine Leukemias'

## Description

### Background of the Invention

### 1. Field of the invention

This invention relates to substituted 5-[(aminoalkyl)amino)imidazo [4,5,1-de]acridin-6-ones and their antineoplastic use, especially for the treatment of leukaemia, and processes for their production.

### 2. Description of the related art

In the paper "5-[(aminoalkyl)aminolimidazo[4,5,1-de)acridin-6-ones as a novel class of antineoplastic agents. Synthesis and Biological Activity", J. Med. Chem. 33, 49-52 (1990), W.M. Cholody, S. Martelli, J. Paradziej-Lukowicz and J. Konopa have briefly reviewed tricyclic ring antineoplastic agents and have described new compounds recited in the title. A later paper, by W. Cholody et. al., J. Med. Chem. 33, 2852-2856 (1990), entitled "8-substituted 5-[(aminoalkyl)amino]-6H-v-triazolo[5,4,1-de]acridin-6-ones as potential antineoplastic agents. Synthesis and Biological Activity", describes these compounds, which differ by the replacement of an optionally substituted imidazo C-atom by a triazolo N-atom (unsubstituted) and optionally by the introduction of an 8-substituent selected from nitro, chloro, methyl, methoxy and hydroxy. The results are described as clearly indicating that high antineoplastic activity of those triazolo ring compounds is related to the A ring hydroxylation. However, structure-activity relationships in relation to in vivo evaluation against murine leukaemia remained unclear. It is, therefore, still a problem to find other compounds with high antineoplastic, especially antileukaemic, activity.

### Summary of the Invention

The present invention provides compounds of formula I: 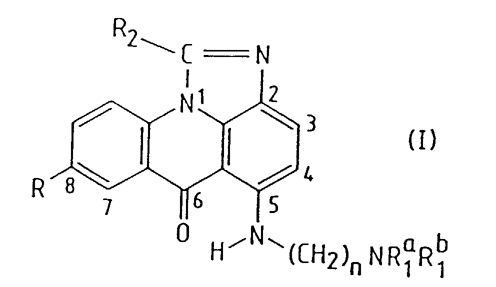 in which:
R represents: -OH or -OR' wherein R' represents C₁-C₆ alkyl, e.g. methyl,
R₁^{a} and R₁^{b}, which may be identical or different, represent hydrogen or C₁-C₆ alkyl, e.g. methyl or ethyl, which is unsubstituted or is substituted by a hydroxyl, an amino, a N'-alkylamino or a N',N'-dialkylamino group, in which the or each alkyl group has 1 - 4 carbon atoms, for example in the substituents: 2-hydroxyethyl, 2-aminoethyl, 2-(N'-alkylamino)ethyl and 2-(N',N'-dialkylamino)ethyl, n is 2-5 and
R₂ represents hydrogen, or straight chain C₁₋₄ alkyl, in the form of the free bases, their pharmaceutically acceptable acid addition salts or N-oxides thereof.

### Description of the preferred embodiments

R₁^{a} and R₁^{b} are normally identical and represent C₁-C₆ alkyl groups, especially C₁-C₃ alkyl, such as methyl or ethyl, n typically being 2 or 3.

The compounds of formula I in which R₁^{a} = R₁^{b} = methyl or ethyl, R₂ = hydrogen or methyl and n is 2 or 3 are of particular interest in treating leukaemia. Of these compounds, those of most interest are those in which:

| | | | | |
|---|---|---|---|---|
| Ex. 9 | R = -OH; | R₁^{a} = R₁^{b} = - CH₃; | R₂ = H; | n = 2. |
| Ex. 10 | R = -OH; | R₁^{a} = R₁^{b} = - CH₃; | R₂ = CH₃; | n = 2. |
| Ex. 11 | R = -OH; | R₁^{a} = R₁^{b} = - CH₂CH₃; | R₂ = H; | n = 2. |
| Ex. 12 | R = -OH; | R₁^{a} = R₁^{b} = - CH₂CH₃; | R₂ = CH₃; | n = 2. |
| Ex. 14 | R = -OH; | R₁^{a} = R₁^{b} = - CH₃; | R₂ = CH₃; | n = 3. |
| Ex. 15 | R = -OH; | R₁^{a} = R₁^{b} = - CH₂CH₃; | R₂ = H; | n = 3. |
| Ex. 16 | R = -OH; | R₁^{a} = R₁^{b} = - CH₂CH₃; | R₂ = CH₃; | n = 3. |

The methyl ethers are generally less active, but among them the compounds in which n = 2, R₂ = H and R₁^{a} = R₁^{b} = CH₃ or C₂H₅, (Examples 1 and 2) are the most preferred.

Addition salts which are generally pharmaceutically acceptable, may be of an organic or inorganic acid. Examples of suitable acids for salt formation are: hydrochloric, sulfuric, phosphoric, acetic, citric, malonic, ascorbic, maleic, methanesulfonic, lactic, gluconic, glucuronic, and the like. Usually the compound I is present in the form of a hydrochloride, which could be a mono-, di- or tri- salt or any mixture thereof. It can also be hydrated to variable extents.

It is known that N-oxides of chemotherapeutic anthraquinones having tertiary amino groups are useful as pro-drugs for antineoplastic therapy, being bioreductively activated within neoplastic tissue to form the active compound: see UK Patent Specification 2 237 283A (NRDC). Mild oxidation, under conditions which will not cause disruption of the imidazole ring, can be used to make N-oxides, of the imidazole N-atom or of a tertiary amine group present when R₁^{a} and R₁^{b} are not hydrogen atoms.

Compounds of formula I (in which R₂ represents hydrogen or a said alkyl group) may be produced as free bases or salts by treating a compound of formula II, optionally in the form of an acid addition salt thereof, 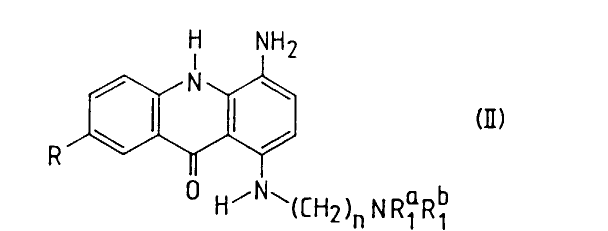 respectively with formic acid or a compound of formula R₂CON(CH₃)₂, preferably at elevated temperature, typically at reflux in the absence of added solvent. Salts can readily be converted to free bases and free bases to salts or N-oxides by methods known per se.

Compounds of formula II, optionally in the form of acid addition salts, may be produced from compounds of formula III by treatment thereof to reduce the nitro group to the corresponding amino group:

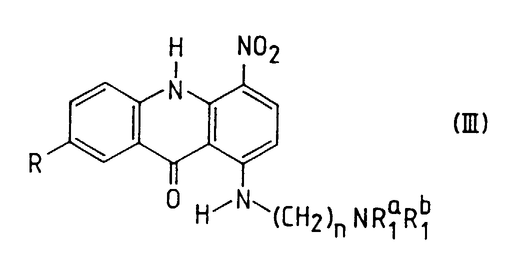

Compounds of formula III can be prepared as described in European Patent Application EP-A 145 226 (Warner-Lambert Company) or procedures analogous thereto. The reduction of the nitro group is preferably carried out by hydrazine hydrate, suitably in the presence of a catalyst, e.g. Raney Nickel, in a polar solvent such as tetrahydrofuran (THF). The intermediates II thus obtained are generally extremely unstable to oxygen, especially in those compounds wherein n represents 3 and are usually used as starting materials for conversion to compounds of formula I in the form of acid addition salts, for example hydrochlorides.

Two methods are generally used for isolation of the final products. In the case of methoxy derivatives, the products may be extracted with benzene or chloroform from the reaction mixture after rendering the mixture alkaline and next transformed into dihydrochlorides. The hydroxy compounds may instead be isolated as hydrochloride salts directly from the reaction mixture after acidification with HCl.

Compounds of formula I are of interest for the treatment or prophylaxis of neoplasms (a term used herein to refer to any malignant tissue growth and therefore to include cancers and leukaemias), especially lymphoblastic leukaemias. All the compounds of the invention tested appear to have antileukaemic activity in the in vitro and/or in vivo tests reported hereinafter (see Table III). In vivo tests of selected compounds of the invention against tumours in animals have shown that the compounds of Examples 11 and 12 are active against the MT-3 human mammary carcinoma and the compounds of Examples 12 and 15 against colon 38 adenocarcinoma and B16 melanoma. In vitro data shows that compounds of Examples 10 and 16 have potent activity against renal cancer cell lines.

Accordingly, in a further aspect the present invention comprises a compound of formula I for use in therapy and, in a yet further aspect of the present invention, comprises the use of a compound of formula I for the manufacture of a medicament useful in antineoplastic treatment or prophylaxis.

The dosage form and amount can be readily established by reference to known antineoplastic treatment or prophylactic regimens. In general, however, the dosage of the compound of formula I usually lies within the range about 0.1mg to about 50mg/kg, preferably 0.5mg to 10mg/kg.

The compound of formula I can be administered alone or in connection with, one or more pharmaceutically acceptable diluents or carriers therefor, and optionally, any other ingredients which may be therapeutic per se, synergistic with the compound of formula I, or both. Carrier(s) and diluents are, of course, pharmaceutically acceptable and compatible with the other ingredients of the formulation.

Formulations suitable for oral, rectal, topical or parenteral (including subcutaneous, intramuscular and intravenous) administration are included. Unit dosage forms may be prepared by known methods. All methods generally include the step of bringing the active compound into association with a carrier or diluent, as a suspension or solution, and optionally one or more accessory ingredients, e.g. buffers, flavouring agents, binders, surface active agents, thickeners, anti-caking agents, lubricants and preservatives (including antioxidants).

Formulations of the present invention suitable for oral administration may be presented as capsules, cachets, tablets or lozenges, powder or granules; or a suspension. Tablets may be prepared by compressing the active compound in a free-flowing form such as a powder or granules, optionally mixed with conventional additives. A syrup may be made by adding the active compound to a concentrated, aqueous solution of a sugar, for example sucrose, optionally containing conventional additives. Formulations suitable for parenteral administration conveniently comprise a sterile aqueous preparation of the active compound which is preferably made isotonic.

The present invention is illustrated by the following Examples:-

### EXAMPLES

### General Procedure

Compounds of formula I, the subject of Examples 1 to 16, are produced by the route outlined in Scheme 1.

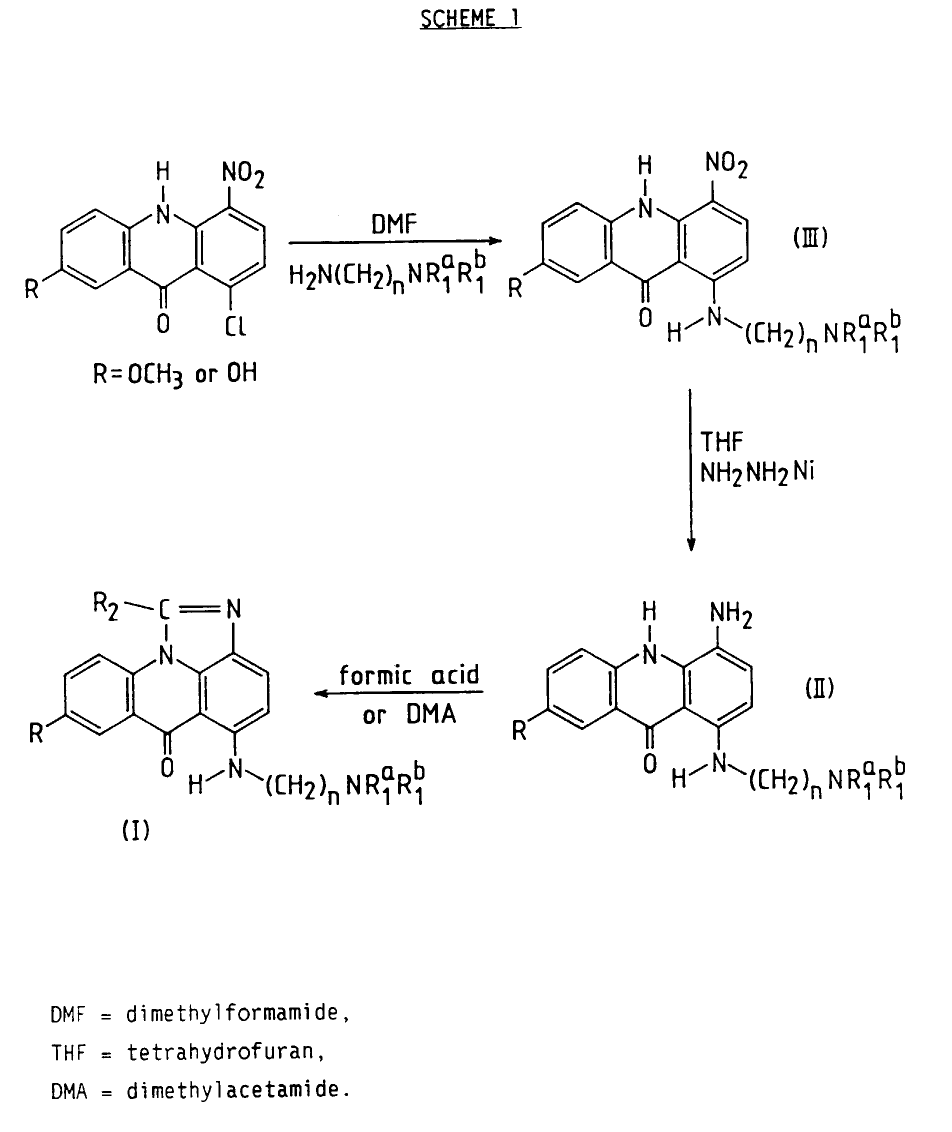

In the following Examples melting points were taken on a Buchi 510 capillary melting points apparatus and are uncorrected. ¹H NMR spectra were recorded on a Varian (Registered Trade Mark) VXR-300 spectrometer operating at 300 MHz. Chemical shifts are reported as δ units in ppm downfield from internal tetramethylsilane. NMR abbreviations used are as follows: br(broad), s(singlet), d(doublet), t(triplet), qu(quartet), qt(quintet), m(multiplet), ex(exchangeable with deuterium oxide). Quartets which by addition of deuterium oxide are transformed into triplets are labeled with *. Single frequency decoupling was utilized to assign specific protons. Coupling constants are given in Hz. Microanalytical results, indicated by atomic symbols, are within ±0.4% of the theoretical values.

### EXAMPLE 1

### A. 1-[[2-(Diethylamino)ethyl]amino]-7-methoxy-4-nitro-9(1OH)-acridinone.

A mixture of 4.57g (0.015 mol) l-chloro-7-methoxy-4-nitro-9(1OH)-acridinone, 25 ml DMF and 7.00g (0.06 mol) 2-diethylaminoethylamine is stirred and heated at 60°C for 30 minutes. 100 ml 40% (v/v) MeOH-water solution is added to the reaction mixture, heated to boiling and after cooling left overnight in a refrigerator. The crystallized product is collected by filtration washed with water (150 ml) and MeOH (50 ml) and dried to give 5.30g. (92%) analytically pure product as yellow needles: mp 178-179°C (lit.mp., European Patent Appl. EP-A 145226, Chem. Abstr. 1985, 103, 215182s., 179-180°C);

### B. Preparation of 7-substituted-4-amino-1-[[(dialkylamino)alkyl]amino]-9(1OH)-acridinone Hydrochloride Salts.

To a mixture of nitro derivatives (0.01 mol), 200 ml THF, and about 2.5g of Raney Ni is added with stirring at room temperature then 2 ml hydrazine monohydrate. Stirring is continued for about 30 minutes. The catalyst is filtered off and washed with THF (50 ml). The filtrate is quickly treated with 10 ml concentrated hydrochloric acid and stirred for 10 minutes. The yellow precipitate obtained is collected and washed with THF.

The product is recrystallized from a solution of MeOH (90%)-dioxane made acidic with HCl (pH~2).

### C. Preparation of 5-[[2-(diethylamino)ethyl]amino]-8-methoxyimidazo[4,5,1-de] acridin-6-one Dihydrochloride.

A mixture of 1.71g (4 mmol) of the product from the procedure of Example 1B and 20 ml 95% formic acid is heated at reflux for 6h. Acid is evaporated and the residue is dissolved in water (100 ml). The solution is made basic (pH 9) by addition of sodium carbonate and product is extracted with chloroform (2 x 100 ml). The organic extracts are dried and evaporated to give a residue which is dissolved in EtOH. The solution is made acidic with HCl and product is crystallized by addition of acetone to give the title product.

### EXAMPLE 2

### Compound I: n=2, R=OCH₃, R₁^{a}=R₁^{b}=CH₃, R₂=H.

The procedures 1A, 1B and 1C of Example 1 are followed but dimethylaminoethylamine is used in place of diethylaminoethylamine in 1A.

### EXAMPLE 3

### Compound I: n=2, R=OCH₃, R₁^{a}=R₁^{b}=CH₃, R₂=CH₃.

The procedures 1A and 1B of Example 1 are followed using dimethylaminoethylamine in place of diethylaminoethylamine. The product is then subjected to the following procedure (designated 3C):

A mixture of 2.14g (5 mmol) hydrochloride and 30 ml DMA is refluxed for 12h, 200 ml water is added to the reaction mixture, made basic with sodium hydroxide and the product is extracted with benzene (2.150 ml). The extracts are evaporated to dryness and the residue is dissolved in methanol-dioxane (1:1) mixture. The solution is made acidic with gaseous HCl and the crystallized product is collected by filtration to give yellow crystals.

### EXAMPLE 4

### Compound I: n=2, R=OCH₃, R₁^{a}=R₁^{b}=CH₂CH₃, R₂=CH₃.

The procedures 1A and 1B of Example 1 are followed and the product is subjected to procedure 3C.

### EXAMPLE 5

### Compound I: n=3, R=OCH₃, R₁^{a}=R₁^{b}=CH₃, R₂=H.

The procedures 1A, 1B and 1C of Example 1 are followed but dimethylaminopropylamine is used in place of diethylaminoethylamine in procedure 1A.

### EXAMPLE 6

### Compound I: n=3, R=OCH₃, R₁^{a}=R₁^{b}=CH₃, R₂=CH₃.

The procedure of Example 5 is followed but procedure 3C replaces procedure 1C.

### EXAMPLE 7

### Compound I: n=3, R=OCH₃, R₁^{a}=R₁^{b}=CH₂CH₃, R₂=H.

The procedures 1A, 1B and 1C of Example 1 are followed but diethylaminopropylamine is used in place of diethylaminoethylamine in procedure 1A.

### EXAMPLE 8

### Compound I: n=3, R=OCH₃, R₁^{a}=R₁^{b}=CH₂CH₃, R₂=CH₃.

The procedure of Example 7 is followed but procedure 3C replaces procedure 1C.

### EXAMPLE 9

### Compound I: n=2, R=OH, R₁^{a}=R₁^{b}=CH₃, R₂=H.

The procedure of Example 1 is followed but dimethylaminoethylamine is used in place of diethylaminoethylamine and 1-chloro-7-hydroxy-4-nitro-9(1OH)-acridone is used in place of 1-chloro-7-methoxy-4-nitro-9(1OH)-acridone in procedure 1A and procedure 1C is replaced by the following, designated 9C:

A mixture of 5 mmol of dihydrochloride salt and 20 ml of 95% formic acid is refluxed for 8h. Formic acid is evaporated and the residue is dissolved on heating in methanol. 3 ml conc. hydrochloric acid is added to the hot solution and the product is crystallized by addition of acetone. The product is collected by filtration and recrystallized from a methanol-acetone mixture.

### EXAMPLE 10

### Compound I: n=2, R=OH, R₁^{a}=R₁^{b}=CH₃, R₂=CH₃.

The procedure of Example 9 is followed but the following procedure, designated 10C, replaces 9C:

A mixture of 5 mmol of dihydrochloride salt and 25 ml of DMA is refluxed for 12h. About 20 ml of the solvent is evaporated, 100 ml acetone is added to the residue and the solution is acidified with gaseous HCl. The precipitated product is collected by filtration and washed with acetone. Crude product is recrystallized (if necessary twice) from methanol-acetone to give the respective dihydrochloride salt.

### EXAMPLE 11

### Compound I: n=2, R=OH, R₁^{a}=R₁^{b}=CH₂CH₃, R₂=H.

The procedure of Example 9 is followed but diethylaminoethylamine is used in place of dimethylaminoethylamine.

### EXAMPLE 12

### Compound I: n=2, R=OH, R₁^{a}=R₁^{b}=CH₂CH₃, R₂=CH₃.

The procedure of Example 10 is followed but diethylaminoethylamine is used in place of dimethylaminoethylamine.

### EXAMPLE 13

### Compound I: n=3, R=OH, R₁^{a}=R₁^{b}=CH₃, R₂=H.

The procedure of Example 9 is followed but dimethylaminopropylamine is used in place of dimethylaminoethylamine.

### EXAMPLE 14

### Compound I: n=3, R=OH, R₁^{a}=R₁^{b}=CH₃, R₂=CH₃.

The procedure of Example 13 is followed but procedure 10C replaces procedure 9C.

### EXAMPLE 15

### Compound I: n=3, R=OH, R₁^{a}=R₁^{b}=CH₂CH₃, R₂=H.

The procedure of Example 9 is followed but diethylaminopropylamine is used in place of dimethylaminoethylamine.

### EXAMPLE 16

### Compound I: n=3, R=OH, R₁^{a}=R₁^{b}=CH₂CH₃, R₂=CH₃.

The procedure of Example 15 is followed but procedure 10C replaces procedure 9C.

### Intermediates

Melting points, yields and molecular formulae of starting compounds (III) and intermediates (II) are set forth in Table I.

**TABLE I**

| 1-Substituted 4-Nitro-9(1OH)-acridinones (III) and 1-Substituted 4-Amino-7-methoxy-9-(1OH)-acridinones (II) | | | | | | |
|---|---|---|---|---|---|---|
| Compd | n | R | R₁^{a} = R₁^{b} | mp,°C | yield,% | molecular formula* |
| (III) | 2 | OCH₃ | CH₃ | 242-243 | 96 | C₁₈H₂₀N₄O₄ |
| " | 2 | OCH₃ | CH₂CH₃ | 178-179 | 92 | C₂₀H₂₄N₄O₄ |
| " | 3 | OCH₃ | CH₃ | 165-166 | 94 | C₁₉H₂₂N₄O₄ |
| " | 3 | OCH₃ | CH₂CH₃ | 153-154 | 97 | C₂₁H₂₆N₄O₄ |
| " | 2 | OH | CH₃ | 258-260 | 90 | C₁₇H₁₈N₄O₄ |
| " | 2 | OH | CH₂CH₃ | 227-229 | 94 | C₁₉H₂₂N₄O₄ |
| " | 3 | OH | CH₃ | 213-214 | 82 | C₁₈H₂₀N₄O₄ |
| " | 3 | OH | CH₂CH₃ | 208-210 | 86 | C₂₀H₂₄N₄O₄ |
| (II) | 2 | OCH₃ | CH₃ | 240-243 dec. | 79 | C₁₈H₂₂N₄O₂.2HCl |
| " | 2 | OCH₃ | CH₂CH₃ | 227-231 dec. | 74 | C₂₀H₂₆N₄O₂.2HCl |
| " | 3 | OCH₃ | CH₃ | 232-235 dec. | 80 | C₁₉H₂₄N₄O₂.2HCl |
| " | 3 | OCH₃ | CH₂CH₃ | 180-185 dec. | 84 | C₂₁H₂₈N₄O₂.3HCl |

| | | | | | | |
|---|---|---|---|---|---|---|
| *The analyses are within ±0.4% of the theoretical values for C, H and N. | | | | | | |

### Final Compounds

Melting points, yields and molecular formulae of compounds of the invention are set forth in Table II, with reference to formula I. It will be appreciated that such compounds can readily be prepared as the free bases or as other acid addition salts and/or hydrates and therefore that the disclosure of Table II is not to be construed as limited by such particular forms of compound. NMR spectra of certain compounds are shown below.

### Compound I: R=OCH₃, R₁^{a}=R₁^{b}=CH₂CH₃, R₂=H, n=2. (Ex.3)

¹H NMR (free base)(Me₂SO-d₆) δ 9.13(s, 1H,Cl-**H**), 8.98(t,1H, ex,-N**H**-CH₂-), 8.36(d,1H,J=9.1,C10-**H**), 7.98(d,1H,J=8.9,C3-**H**), 7.79(d,1H,J=3.0,C7-**H**), 7.52(dd,1H,J=9.1,J=3.0,C9-**H**), 6.80(d,1H, J=8.9,C4-**H**), 3.92(s,3H,-OC**H**₃), 3.42(qu*,2H,-NH-C**H**₂-CH₂-), 2.73(t, 2H,-CH₂-C**H**₂-NEt₂), 2.58(qu,4H,-N(C**H**₂-CH₃)₂), 1.02(t,6H,-N(CH₂-C**H**₃)₂).

### Compound I: R=OCH₃, R₁^{a}=R₁^{b}=CH₂CH₃, R₂=CH₃, n=2. (Ex.4)

¹H NMR (free base) (Me₂SO-d₆) δ 8.98(t,1H,ex,-N**H**-CH₂), 8.12(d,1H,J=9.2,C10-**H**), 7.82(d,1H,J=3.2,C7-**H**), 7.80(d,1H,J=8.8, C3-**H**), 7.43(dd,1H,J=9.2,J=3.2,C9-**H**), 6.70(d,1H,J=8.8,C4-**H**), 3.91(s,3H,-OC**H**₃), 3.38(qu*,2H,-NH-C**H**₂-CH₂-), 3.00(s,3H,Cl-C**H**₃), 2.72(t,2H,-CH₂-C**H**₂-NEt₂), 2.58(qu,4H,-N(C**H**₂-CH₃)₂), 1.03(t,6H,-N(CH₂-C**H**₃)₂).

### Compound I: R=OH, R₁^{a}=R₁^{b}=CH₂CH₃, R₂=H, n=2. (Ex.11)

¹H NMR (free base) (Me₂SO-d₆) δ 10.00(s,1H,ex,C8-0**H**), 9.08(s,1H,Cl-**H**), 8.99(t,1H,ex,-N**H**-CH₂-), 8.26(d,1H,J=8.9,C10-**H**), 7.95(d,1H,J=8.8,C3-**H**), 7.72(d,1H,J=2.8,C7-**H**), 7.33(dd,1H,J=8.9, J=2-8,C9-**H**), 6.77(d,1H,J=8.8,C4-**H**), 3.40(qu*,2H,-NH-C**H**₂-CH₂-), 2.70(t,2H,-CH₂-C**H**₂-NEt₂), 2.56(qu,4H,-N(C**H**₂-CH₃)₂), 1.01(t,6H,-N(CH₂-C**H**₃)₂).

### Compound I: R=OH, R₁^{a}=R₁^{b}=CH₂CH₃, R₂=CH₃, n=2. (Ex.12)

¹H NMR (free base) (ME₂SO-d₆) δ 10.00(s,1H,ex,C8-O**H**), 9.02(t,1H,ex,-N**H**-CH₂-), 8.11(d,1H,J=9.1,(C10-**H**, 7.83(d,1H,J=8.8, C3-**H**), 7.79(d,1H,J=2.9,C7-**H**), 7.33(dd,1H,J=9.1,J=2.9,C9-**H**), 6.72(d,1H,J=8.8,C4-**H**), 3.38(qu*,2H,-NH-C**H**₂-CH₂-), 3.02(s,3H,Cl-C**H**₃), 2.72(t,2H,-CH₂-C**H**₂-NEt₂), 2.56(qu,4H,-N(C**H**₂-CH₃)₂), 1.02(t, 6H,-N(CH₂-C**H**₃)₂).

### Compound I: R=OH,R₁^{a}=R₁^{b}=CH₂CH₃R₂=H, n=3. (Ex.15)

¹H NMR (free base) (ME₂SO-d₆) δ 10.02(br s,1H,ex,C8-O**H**), 9.10(s,1H,Cl-**H**), 8.93(t,1H,ex,-N**H**-CH₂-), 8.27(d,1H,J=8.9,C10-**H**), 7.97(d,1H,J=8.8,C3-**H**), 7.73(d,1H,J=2.8,C7-**H**), 7.34(dd,1H,J=8.9, J=2.8,C9-H), 6.81(d,1H,J=8.8,C4-**H**), 3.42(qu*,2H,-NH-C**H**₂-CH₂-), 2.52(t,2H,-CH₂-C**H**₂-NEt₂), 2.48(qu,4H,-N(C**H**₂,CH₃)₂), 1.78(qt,2H,-CH₂-C**H**₂-CH₂-), 0.96(t,6H,-N(CH₂-C**H**₃)₂).

### Compound I: R=OH, R₁^{a}=R₁^{b}=CH₂CH₃, R₂=CH₃, n=3. (Ex.16)

¹H NMR (free base) (Me₂SO-d₆) δ 10.0(br s,1H,ex,C8-O**H**), 8.91(t,1H,ex,-N**H**-CH₂-), 8.08(d,1H,J=9.1,C10-**H**), 7.80(d,1H,J=8.8, C3-**H**), 7.77(d,1H,J=3.0,C7-**H**), 7.32(dd,1H,J=9.1,J=3.0,C9-**H**), 6.70(d,1H,J=8.8,C4-**H**), 3.38(qu*,2H,-NH-C**H**₂-CH₂-), 3.00(s,3H,ClC**H**₃), 2.48(m,6H,-CH₂-C**H**₂-N(C**H**₂-CH₃)₂), 1.76(qt,2H,-CH₂-C**H**₂-CH₂-).

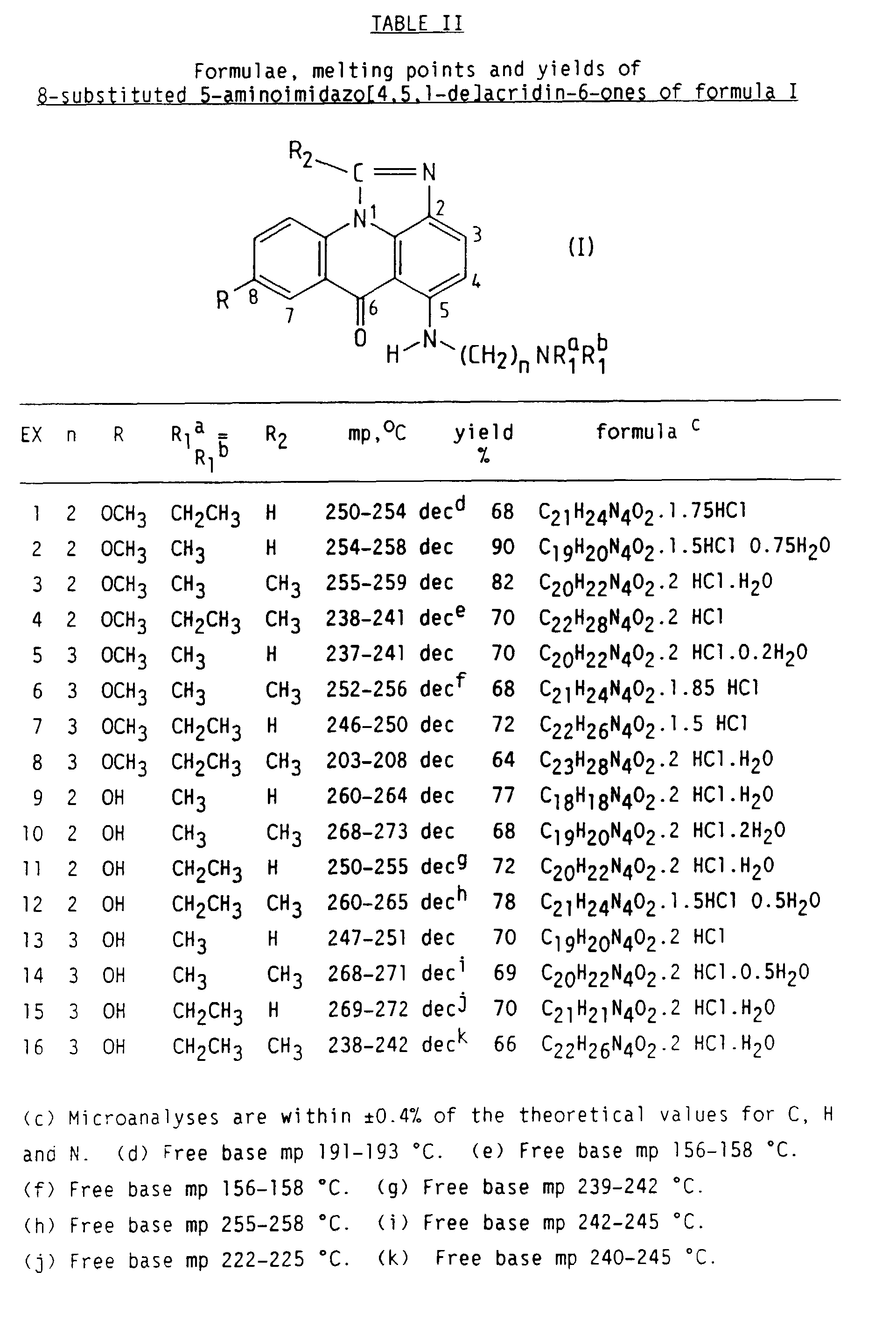

### Biological Tests

### In Vitro Cytoxicity Evaluation

The mouse L1210 leukaemia cells (RPM1, USA) are grown in RPM1 1640 medium supplemented with 5% fetal calf serum and penicillin (1,000,000 units /litre) plus streptomycin (100 mg/litre) in controlled air-5% CO₂ humidified atmosphere at 37°C. L1210 mouse leukemia cells are seeded at a density of 5x10⁴ cells/ml. The tested compounds, dissolved in 50% ethanol, are added, at four different concentrations, to the cell suspensions. The cytotoxic activity (IC₅₀ value) of the tested compounds is defined as their concentrations causing 50% growth inhibition after 48h, measured by cell protein contents and is determined from dose-response curves by the method of J. Konopa, A. Matuszkiewicz, M. Hrabowska, K. Onoszko, Arzneim.-Forsch. 1974, 24, (1971).

### In Vivo Antileukaemic Evaluation

BDF₁ mice are injected ip with 10⁶ P388 lymphotic leukemia cells on day 0 and treated ip on days 1-5 in accordance with the protocols described by the National Cancer Institute : R.I. Geran, et al., Cancer Chemotherapy Reports, Part 3, 3(2), 1-103 (1972) [ISSN=0069-0139]. The mean survival time (MST) for each treatment group (eight mice) is calculated and the percent of T/C was determined by using the following formula:
%T/C=[(MST treated)/(MST control)]x100.

Results of the Cytotoxicity Evaluation and Antileukaemic Evaluation are set forth in Table III:

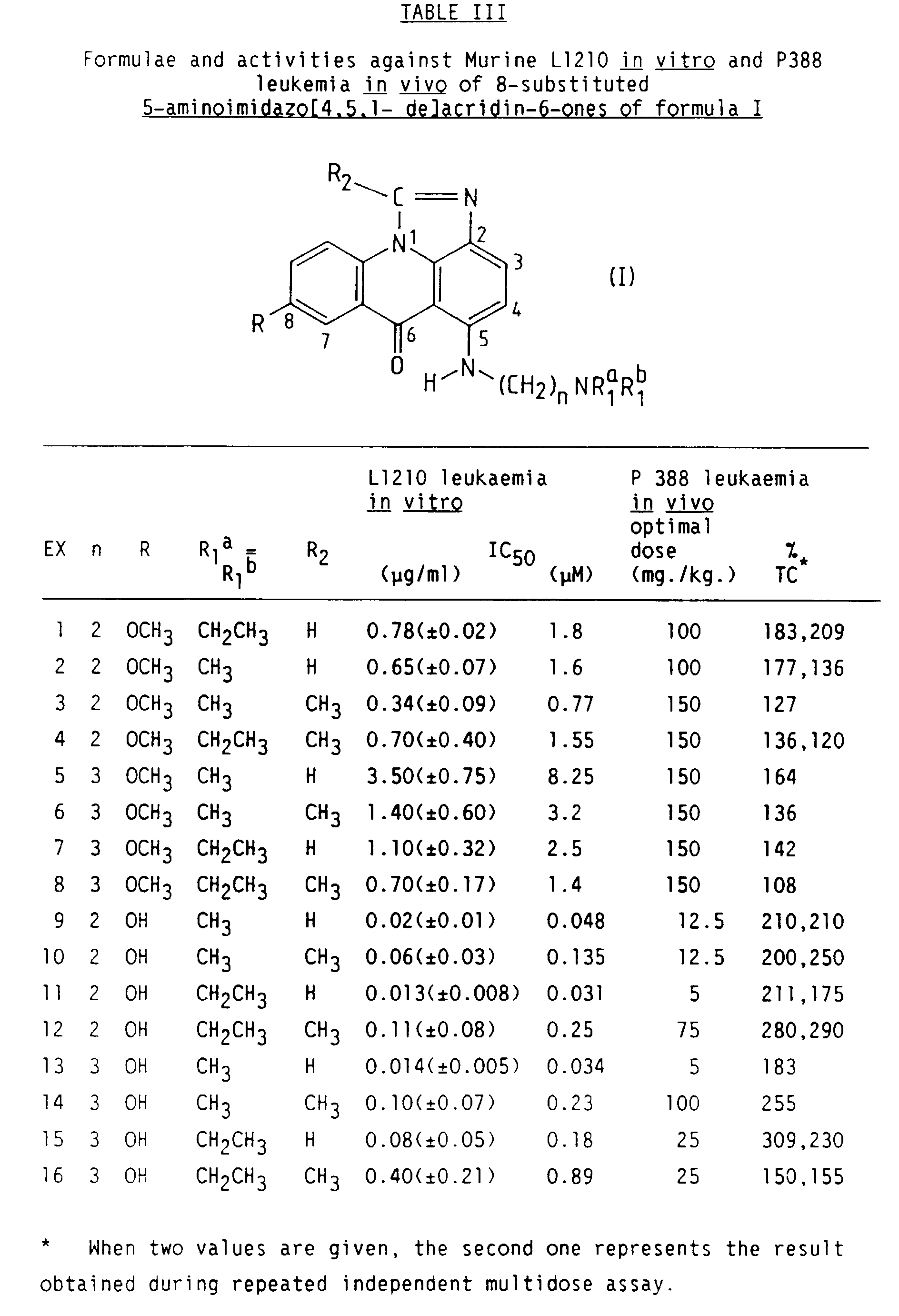

### Free-radical testing

Many anti-cancer compounds exhibit cardiotoxic side effects which are usually ascribed to the liberation of free radicals. Accordingly four representative compounds of the invention, those of Examples 1, 11, 12 and 15, were tested in rat liver microsomes, in comparison with doxorubicin. Whereas 500µM of doxorubicin in NADPH-supplemented rat liver microsomes, under nitrogen purging, gave an electron spin resonance (ESR) signal indicative of the presence of doxorubicin semiquinone free radical, the same molar concentration (500µM) of the four compounds of the invention gave no ESR spectrum over a three hour period.

## Claims (Claims for the following Contracting State(s): AT, BE, CH, DE, DK, FR, IT, LI, LU, NL, PT, SE)

1. Compounds of formula I: 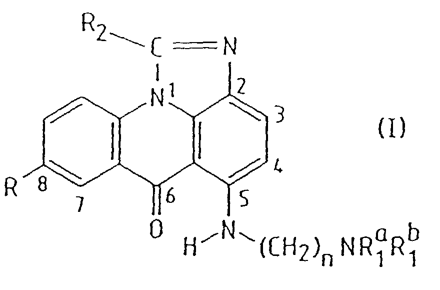 in which:
R represents: -OH or -OR', wherein R' represents C₁-C₆ alkyl,
R₁^{a} and R₁^{b}, which may be identical or different, represent hydrogen or C₁-C₆ alkyl, unsubstituted or substituted by a hydroxyl, an amino, a N'-alkylamino or a N',N'-dialkylamino group, such N'-alkyl groups containing 1-4 carbon atoms,
n is 2-5 and
R₂ represents hydrogen, or straight chain C₁₋₄ alkyl,
in the form of a free base or a pharmaceutically acceptable acid addition salt, or an N-oxide thereof.

2. Compounds according to Claim 1, in which R = -OH, R₁^{a} and R₁^{b} both represent a C₁-C₃ alkyl group and n is 2 or 3.

3. Compounds according to claim 1, in which R = -OH, R₁^{a} = R₁^{b} = ethyl, R₂ = hydrogen or methyl and n is 2.

4. Compounds according to claim 1, in which R = -OH, R₁^{a} = R₁^{b} = ethyl and either (1) R₂ = methyl and n = 2 or (2) R₂ = hydrogen and n = 3.

5. A process for the production of a compound of formula I claimed in claim 1, wherein a compound of formula II, optionally in the form of an acid addition salt thereof, 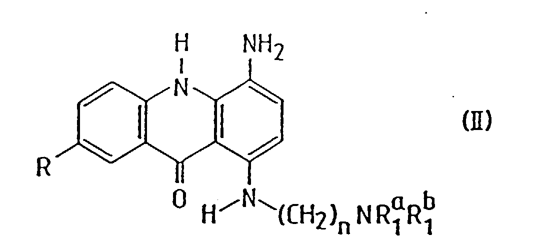 is treated with formic acid or an N,N-dialkylamide of formula R₂CON(CH₃)₂, R₂ representing straight chain C₁₋₄ alkyl, and, if necessary, an acid addition salt is converted to the free base or the free base to a pharmaceutically acceptable acid addition salt or to an N-oxide.

6. A process according to claim 5, in which R = -OH, R₁^{a} = R₁^{b} = ethyl, R₂ = hydrogen or methyl and n is 2.

7. A process according to claim 5, in which R = -OH, R₁^{a} = R₁^{b} = ethyl and either (1) R₂ = methyl and n = 2 or (2) R₂ = hydrogen and n = 3.

8. Compounds according to Claim 3, for use in treating breast cancer.

9. Compounds according to claim 4, for use in treating colonic cancer.

10. A pharmaceutical composition which comprises a compound according to claim 1, 2, 3 or 4, in association with a pharmaceutically acceptable diluent or carrier therefor.

## Claims (Claims for the following Contracting State(s): ES, GR)

1. Process for the preparation of a compound of formula I: 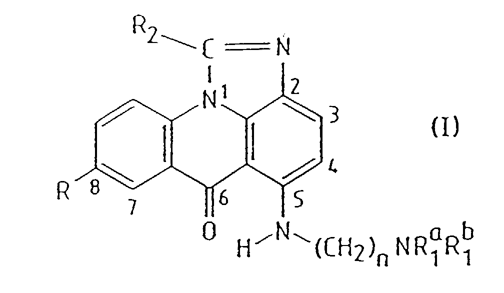 in which:
R represents: -OH or -OR', wherein R' represents C₁-C₆ alkyl,
R₁^{a} and R₁^{b}, which may be identical or different, represent hydrogen or C₁-C₆ alkyl, unsubstituted or substituted by a hydroxyl, an amino, a N'-alkylamino or a N',N'-dialkylamino group, such N'-alkyl groups containing 1-4 carbon atoms,
n is 2-5 and
R₂ represents hydrogen, or straight chain C₁₋₄ alkyl,
in the form of a free base or a pharmaceutically acceptable acid addition salt, or an N-oxide thereof, characterised in that a compound of formula II, optionally in the form of an acid addition salt thereof, 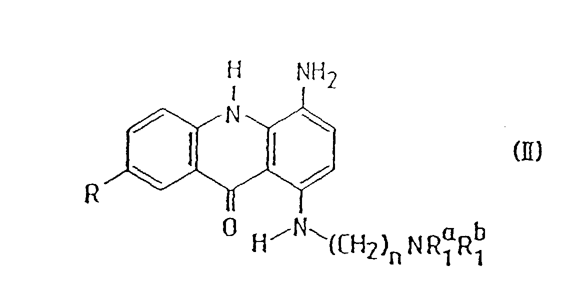 is treated with formic acid or an N,N-dialkylamide of formula R₂CON(CH₃)₂, R₂ representing straight chain C₁₋₄ alkyl, and, if necessary, an acid addition salt is converted to the free base or the free base to a pharmaceutically acceptable acid addition salt or to an N-oxide.

2. Process according to Claim 1, characterised in that a compound of formula (I) in which R = -OH, R₁^{a} and R₁^{b} both represent a C₁-C₃ alkyl group and n is 2 or 3 is prepared.

3. Process according to claim 1, characterised in that a compound of formula (I) in which R = -OH, R₁^{a} = R₁^{b} = ethyl, R₂ = hydrogen or methyl and n is 2 is prepared.

4. Process according to claim 1, characterised in that a compound of formula (I) in which R = -OH, R₁^{a} = R₁^{b} = ethyl and either (1) R₂ = methyl and n = 2 or (2) R₂ = hydrogen and n = 3 is prepared.

## Patentansprüche (Patentansprüche für folgende(n) Vertragsstaat(en): AT, BE, CH, DE, DK, FR, IT, LI, LU, NL, PT, SE)

1. Verbindungen der Formel I: 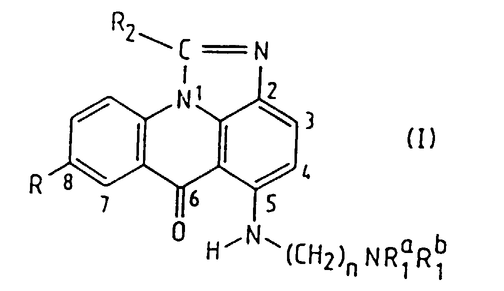 worin:
R für -OH oder -OR' steht, wobei R' C₁-C₆-Alkyl bedeutet,
R₁^{a} und R₁^{b}, welche gleich oder unterschiedlich sein können, Wasserstoff oder C₁-C₆-Alkyl bedeuten, welches unsubstituiert ist oder substituiert ist durch ein Hydroxyl, ein Amino, eine N'-Alkylamino- oder eine N',N'-Dialkylaminogruppe, wobei solche N'-Alkylgruppen 1 bis 4 Kohlenstoffatome besitzen,
n 2 bis 5 ist und
R₂ für Wasserstoff oder für ein geradkettiges C₁₋₄-Alkyl steht,
in Form einer freien Basen oder eines pharmazeutisch annehmbaren Säureadditionssalzes oder eines N-Oxides davon.

2. Verbindungen gemäß Anspruch 1, worin R = -OH ist, R₁^{a} und R₁^{b} beide für eine C₁-C₃-Alkylgruppe stehen und n 2 oder 3 ist.

3. Verbindungen gemäß Anspruch 1, worin R = -OH ist, R₁^{a} = R₁^{b} = Ethyl gilt, R₂ = Wasserstoff oder Methyl ist und n 2 ist.

4. Verbindungen gemäß Anspruch 1, worin R = -OH ist, R₁^{a} = R₁^{b} = Ethyl gilt und entweder (1) R₂ = Methyl und n = 2 sind oder (2) R₂ = Wasserstoffund n = 3 sind.

5. Verfahren zur Herstellung einer Verbindung der Formel I, beansprucht in Anspruch 1, wobei eine Verbindung der Formel II, gegebenenfalls in Form eines Säureadditionssalzes davon, 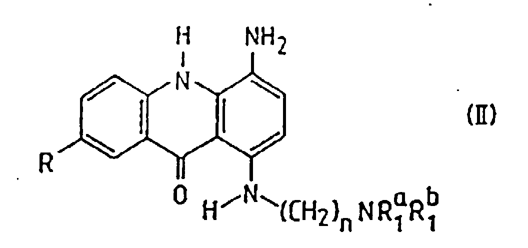 mit Ameisensäure oder einem N,N-Dialkylamid der Formel R₂CON(CH₃)₂ behandelt wird, wobei R₂ für ein geradkettiges C₁₋₄-Alkyl steht, und, sofern erforderlich, ein Säureadditionssalz zu der freien Base oder die freie Base zu einem pharmazeutisch annehmbaren Säureadditionssalz oder zu einem N-Oxid umgewandelt wird.

6. Verfahren gemäß Anspruch 5, worin R = -OH ist, R₁^{a} = R₁^{b} = Ethyl gilt, R₂ = Wasserstoff oder Methyl ist und n 2 ist.

7. Verfahren gemäß Anspruch 5, worin R = -OH ist, R₁^{a} = R₁^{b} = Ethyl gilt und entweder (1) R₂ = Methyl und n = 2 sind oder (2) R₂ = Wasserstoffund n = 3 sind.

8. Verbindungen gemäß Anspruch 3 zur Verwendung bei der Behandlung von Brustkrebs.

9. Verbindungen gemäß Anspruch 4 zur Verwendung bei der Behandlung von Kolonkrebs.

10. Pharmazeutische Zusammensetzung, welche eine Verbindung gemäß Anspruch 1, 2, 3 oder 4 in Verbindung mit einem pharmazeutisch annehmbaren Verdünnungsmittel oder Trägermittel dafür beinhaltet.

## Patentansprüche (Patentansprüche für folgende(n) Vertragsstaat(en): ES, GR)

1. Verfahren zur Herstellung der Verbindung der Formel I: 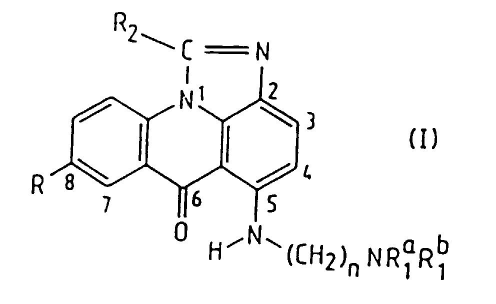 worin:
R für -OH oder -OR' steht, wobei R' C₁-C₆-Alkyl bedeutet,
R₁^{a} und R₁^{b} , welche gleich oder unterschiedlich sein können, Wasserstoff oder C₁-C₆-Alkyl bedeuten, welches unsubstituiert ist oder substituiert ist durch ein Hydroxyl, ein Amino, eine N'-Alkylamino- oder eine N',N'-Dialkylaminogruppe, wobei solche N'-Alkylgruppen 1 bis 4 Kohlenstoffatome besitzen,
n 2 bis 5 ist und
R₂ für Wasserstoff oder für ein geradkettiges C₁₋₄-Alkyl steht,
in Form einer freien Basen oder eines pharmazeutisch annehmbaren Säureadditionssalzes oder eines N-Oxides davon, dadurch gekennzeichnet, daß eine Verbindung der Formel II, gegebenenfalls in Form eines Säureadditionssalzes davon, 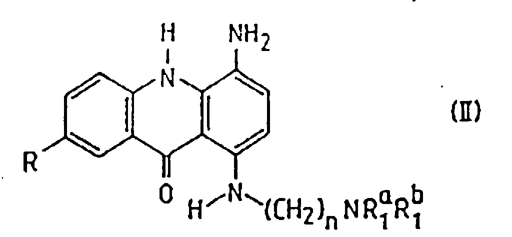 mit Ameisensäure oder einem N,N-Dialkylamid der Formel R₂CON(CH₃)₂ behandelt wird, wobei R₂ für ein geradkettiges C₁₋₄-Alkyl steht, und, sofern erforderlich, ein Säureadditionssalz zu der freien Base oder die freie Base zu einem pharmazeutisch annehmbaren Säureadditionssalz oder zu einem N-Oxid umgewandelt wird.

2. Verfahren gemäß Anspruch 1, dadurch gekennzeichnet, daß eine Verbindung der Formel (I), in der R = -OH ist, R₁^{a} und R₁^{b} beide für eine C₁-C₃-Alkylgruppe stehen und n 2 oder 3 ist, hergestellt wird.

3. Verfahren gemäß Anspruch 1, dadurch gekennzeichnet, daß eine Verbindung der Formel (I), in der R = -OH ist, R₁^{a} = R₁^{b} = Ethyl gilt, R₂ = Wasserstoff oder Methyl ist und n 2 ist, hergestellt wird.

4. Verfahren gemäß Anspruch 1, dadurch gekennzeichnet, daß eine Verbindung der Formel (I), in der R = -OH ist, R₁^{a} = R₁^{b} = Ethyl gilt und entweder (1) R₂ = Methyl und n = 2 sind oder (2) R₂ = Wasserstoffund n = 3 sind, hergestellt wird.

## Revendications (Revendications pour l'(les) Etat(s) contractant(s) suivant(s): AT, BE, CH, DE, DK, FR, IT, LU, NL, PT, SE)

1. Composés de formule I: 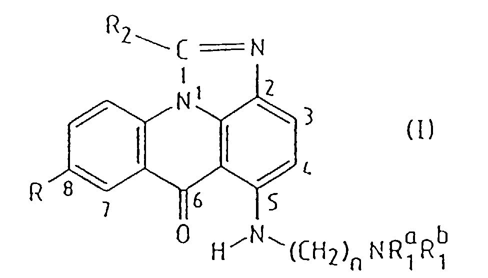 dans laquelle:
R représente: -OH ou -OR', où R' représente un groupe alkyle en C₁-C₆,
R₁^{a} et R₁^{b}, qui peuvent être identiques ou différents, représentent un atome d'hydrogène ou un groupe alkyle en C₁-C₆, non substitué ou substitué par un groupe hydroxy, amino, N'-alkylamino ou N',N'-dialkylamino, ces groupes N'-alkyles comportant de 1 à 4 atomes de carbone,
n est 2 à 5 et
R₂ représente un atome d'hydrogène ou une chaîne alkyle linéaire en C₁₋₄,
sous forme d'une base libre ou d'un sel d'addition acide pharmaceutiquement acceptable ou de N-oxyde de ceux-ci.

2. Composés selon la revendication 1, dans lesquels R = -OH, R₁^{a} et R₁^{b} représentent tous deux un groupe alkyle en C₁-C₃ et n est 2 ou à 3.

3. Composés selon la revendication 1, dans lesquels R = -OH, R₁^{a} = R₁^{b} = éthyle, R₂ = hydrogène ou un groupe méthyle et n = 2.

4. Composés selon la revendication 1, dans lesquels R = -OH, R₁^{a} = R₁^{b} = éthyle et soit (1) R₂ = méthyle et n =2, soit (2) R₂ = hydrogène et n = 3.

5. Procédé pour la production d'un composé de formule I tel que revendiqué selon la revendication 1, dans lequel on traite un composé de formule II, éventuellement sous forme d'un sel d'addition acide de celui-ci, 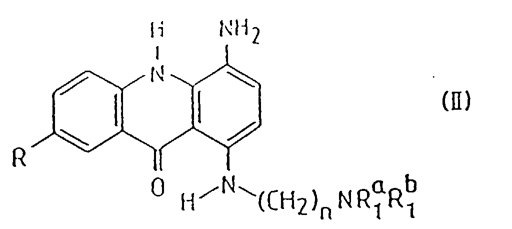 avec de l'acide formique ou un N,N-dialkylamide de formule R₂CON(CH₃)₂, R₂ représentant une chaîne alkyle linéaire en C₁₋₄ et, si nécessaire, on transforme le sel d'addition acide en base libre ou la base libre en un sel d'addition acide pharmaceutiquement acceptable ou en un N-oxyde.

6. Procédé selon la revendication 5, dans lequel R = -OH, R₁^{a} = R₁^{b} = éthyle, R₂ = hydrogène ou méthyle et n = 2.

7. Procédé selon la revendication 5, dans lequel R = -OH, R₁^{a} = R₁^{b} = éthyle et soit (1) R₂ = méthyle et n =2, soit (2) R₂ = hydrogène et n = 3.

8. Composés selon la revendication 3, destinés à être utilisés dans le traitement du cancer du sein.

9. Composés selon la revendication 4, destinés à être utilisés dans le traitement du cancer du colon.

10. Composition pharmaceutique comprenant un composé selon la revendication 1, 2, 3 ou 4, associé avec un diluant ou un véhicule pharmaceutiquement acceptable pour celui-ci,

## Revendications (Revendications pour l'(les) Etat(s) contractant(s) suivant(s): ES, GR)

1. Procédé pour la préparation d'un composé de formule I: 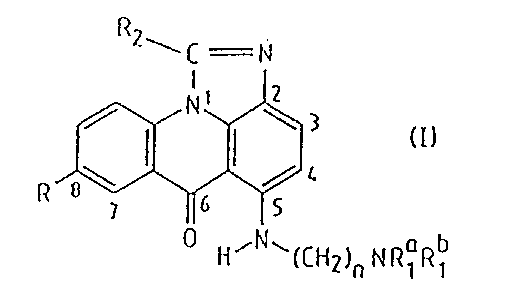 dans laquelle:
R représente: -OH ou -OR', où R' représente un groupe alkyle en C₁-C_{6,}
R₁^{a} et R₁^{b}, qui peuvent être identiques ou différents, représentent un atome d'hydrogène ou un groupe alkyle en C₁-C₆, non substitué ou substitué par un groupe hydroxy, un groupe amino, un groupe N'-alkylamino ou un groupe N',N'-dialkylamino, ces groupes N'-alkyles comportant 1 à 4 atomes de carbone,
n est 2 à 5 et
R₂ représente un atome d'hydrogène ou une chaîne alkyle linéaire en C_{1-4,}
sous forme d'une base libre ou d'un sel d'addition acide pharmaceutiquement acceptable ou de N-oxyde de ceux-ci, caractérisé en ce qu'on traite un composé de formule II, éventuellement sous forme d'un sel d'addition acide de celui-ci, 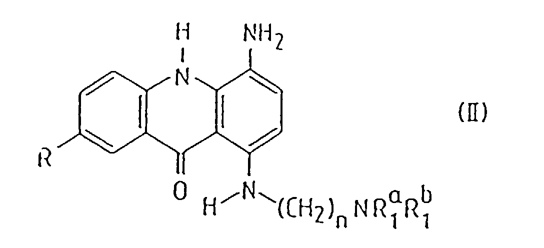 avec de l'acide formique ou un N,N-dialkylamide de formule R₂CON(CH₃)₂, R₂ représentant une chaîne alkyle linéaire en C₁₋₄ et, si nécessaire, on transforme le sel d'addition acide en base libre ou la base libre en un sel d'addition acide pharmaceutiquement acceptable ou en un N-oxyde.

2. Procédé selon la revendication 1, caractérisé en ce qu'on prépare un composé de formule (I) dans lequel R= -OH, R₁^{a} et R₁^{b} représentent tous deux un groupe alkyle en C₁-C₃ et n est 2 ou 3.

3. Procédé selon, la revendication 1, caractérisé en ce qu'on prépare un composé de formule (I) dans lequel R = -OH, R₁^{a} = R₁^{b} = éthyle, R₂ = hydrogène ou méthyle et n = 2.

4. Procédé selon la revendication 1, caractérisé en ce qu'on prépare un composé de formule (I) dans lequel R= -OH, R₁^{a} = R₁^{b} = éthyle et soit (1) R₂ = méthyle et n =2, soit (2) R₂ = hydrogène et n = 3.
